(19) **Europäisches Patentamt European Patent Office Office européen des brevets**

(11) **EP 3 733 607 A1**

(12) **EUROPEAN PATENT APPLICATION**
published in accordance with Art. 153(4) EPC

(43) Date of publication:
**04.11.2020 Bulletin 2020/45**

(51) Int Cl.:
**C01G 23/053** (2006.01)    **A61K 8/29** (2006.01)
**A61Q 1/00** (2006.01)

(21) Application number: **18893702.3**

(22) Date of filing: **27.12.2018**

(86) International application number:
**PCT/JP2018/048037**

(87) International publication number:
**WO 2019/131830 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2017 JP 2017253654**

(71) Applicant: **SUMITOMO OSAKA CEMENT CO., LTD.**
**Chiyoda-ku**
**Tokyo 102-8465 (JP)**

(72) Inventors:
• **YAKUBO Teppei**
 **Tokyo 102-8465 (JP)**
• **ITO Naoko**
 **Tokyo 102-8465 (JP)**
• **ITAGAKI Tetsuro**
 **Tokyo 102-8465 (JP)**

(74) Representative: **Cabinet Laurent & Charras**
**Le Contemporain**
**50 Chemin de la Bruyère**
**69574 Dardilly Cedex (FR)**

(54) **TITANIUM OXIDE POWDER, AND DISPERSION LIQUID AND COSMETIC USING SAID POWDER**

(57) The titanium oxide powder of the present invention is a titanium oxide powder which has a BET specific surface area of 5 $m^2$/g or more and 15 $m^2$/g or less, and contains octahedral-shaped particles, wherein each octahedral-shaped particle thereof has line segments each of which connects two apexes which face each other and has a maximum value of the line segments, an average value of the maximum values is 300 nm or higher and 1000 nm or lower, and a value (D90/D10) is 1 or higher and 3 or lower, wherein the value is obtained by dividing a value (D90), which corresponds to 90% in a cumulative number percentage of the maximum values of line segments each of which connects two apexes which face each other, by a value (D10) which corresponds to 10% in the cumulative number percentage.

FIG. 1

## Description

Technical Field

The present invention relates to a titanium oxide powder suitable for cosmetics, and a dispersion and cosmetics using the same.

Priority is claimed on Japanese Patent Application No. 2017-253654, filed on December 28, 2017, the content of which is incorporated herein by reference.

Background Art

Titanium oxide particles have excellent light reflection characteristics, ultraviolet ray shielding characteristics, and a concealing ability. Therefore, titanium oxide particles having submicron size to micron size are used for base makeup cosmetics such as foundations.

As titanium oxide particles suitable for cosmetics, for example, spherical anatase-type titanium oxide particles having an average particle diameter of 0.1 $\mu$m to 5 $\mu$m have been proposed (for example, see Patent Literature 1) .

In addition, spherical rutile-type titanium oxide particles which is obtained by accumulating spherical primary particles and which have an apparent average particle diameter of 100 nm or higher have been proposed (for example, see Patent Literature 2).

Citation List

Patent Literature

[Patent Literature No. 1] Japanese Laid-open Patent Publication No. 2013-28563
[Patent Literature No. 2] Japanese Laid-open Patent Publication No. 2014-15340

Summary of Invention

Technical Problem

However, there is a requirement of further improvement for titanium oxide particles capable of decreasing paleness peculiar to titanium oxide particles while having a concealing ability and a feeling of transparency.

The present invention has been made in view of the above circumstances, and an object of the present invention is to provide a titanium oxide powder which is capable of decreasing paleness peculiar to titanium oxide particles while having a concealing ability and a feeling of transparency in a case of being applied to the skin and of which a feeling of squeaky is suppressed, and a dispersion and cosmetics using the same.

Solution to Problem

The titanium oxide powder of the present invention is a titanium oxide powder, which has a BET specific surface area of 5 m$^2$/g or more and 15 m$^2$/g or less, and contains octahedral-shaped particles, wherein each octahedral-shaped particle thereof has line segments each of which connects two apexes which face each other and has a maximum value of the line segments, an average value of the maximum values is 300 nm or higher and 1000 nm or lower, and a value (D90/D10) is 1 or higher and 3 or lower, wherein the value is obtained by dividing a value (D90), which corresponds to 90% in a cumulative number percentage of the maximum values of line segments each of which connects two apexes which face each other, by a value (D10) which corresponds to 10% in the cumulative number percentage.

The dispersion of the present invention includes the titanium oxide powder of the present invention and a dispersion medium.

The cosmetics of the present invention contain the titanium oxide powder of the present invention and a cosmetic base.

Advantageous Effects of Invention

According to the titanium oxide powder of the present invention, it is possible to provide a titanium oxide powder which is capable of decreasing paleness peculiar to titanium oxide particles while having a concealing ability and a feeling of transparency in a case of being applied to the skin and of which a feeling of squeaky is suppressed, and a dispersion and cosmetics using the same.

According to the dispersion of the present invention, it is possible to decrease paleness peculiar to titanium oxide particles while having a concealing ability and a feeling of transparency in a case where cosmetics containing the dispersion are applied to the skin and to suppress a feeling of squeaky.

According to the cosmetics of the present invention, it is possible to decrease paleness peculiar to titanium oxide particles while having a concealing ability and a feeling of transparency in a case of being applied to the skin and to suppress a feeling of squeaky.

Brief Description of Drawings

FIG. 1 is a schematic diagram showing a preferred example of octahedral-shaped titanium oxide particles.
FIG. 2 is another schematic diagram showing a preferred example of octahedral-shaped titanium oxide particles.
FIG. 3 is a view showing a scanning electron microscope image of titanium oxide particles of Example 1.
FIG. 4 is a view showing a result of simulating how light scatters in a case where spherical-shaped titanium oxide particles are irradiated with light.
FIG. 5 is a view showing a result of simulating how light scatters in a case where octahedral-shaped titanium oxide particles are irradiated with light.

Description of Embodiments

Preferred embodiments of a titanium oxide powder of the present invention, and a dispersion and cosmetics using the same will be described.

The present embodiments will be described in detail in order to better understand the gist of the invention, and the present invention is not limited unless otherwise specified. Omissions, additions, substitutions, and other changes can be made without departing from the gist of the present invention.

[Titanium oxide powder]

The titanium oxide powder of the present embodiment is a titanium oxide powder, which has a BET specific surface area of $5 m^2/g$ or more and $15 m^2/g$ or less. The titanium oxide powder contains octahedral-shaped particles. Each octahedral-shaped particle of the octahedral-shaped particles has line segments each of which connects two apexes which face each other and has a maximum value of the line segments, and an average value of the maximum values of the octahedral-shaped particles is 300 nm or higher and 1000 nm or lower. In the octahedral-shaped particles, a value (D90/D10) is 1 or higher and 3 or lower, wherein the value is obtained by dividing a value (D90), which corresponds to 90% in a cumulative number percentage of the maximum values of line segments each of which connects two apexes which face each other, by a value (D10) which corresponds to 10% in the cumulative number percentage. Hereinafter, "octahedral-shaped particles in which each octahedral-shaped particle thereof has line segments each of which connects two apexes which face each other and has a maximum value of the line segments, and an average value of the maximum values is 300 nm or higher and 1000 nm or lower" is referred to as "octahedral-shaped particles" in some cases.

The average value of the maximum values means an average value obtained from a plurality of octahedral-shaped titanium oxide particles, wherein each octahedral-shaped titanium oxide particle has a plurality of line segments each of which connects two apexes which face each other and has a maximum value of lengths as the longest line segment. In addition, the line segment connecting two apexes which face each other exists inside the particle but not a surface of the particle.

(Specific surface area)

A BET specific surface area of the titanium oxide powder of the present embodiment is $5 m^2/g$ or more and $15 m^2/g$ or less, and preferably $5 m^2/g$ or more and $13 m^2/g$ or less.

A case where the BET specific surface area of the titanium oxide powder is $5 m^2/g$ or more and $15 m^2/g$ or less is advantageous from the viewpoint of further decreasing paleness peculiar to titanium oxide particles while having a concealing ability and a feeling of transparency. Furthermore, in a case where the BET specific surface area of the titanium oxide powder is less than $5 m^2/g$, a feeling of transparency is deteriorated due to light scattering. On the other hand, in a case where the BET specific surface area of the titanium oxide powder is greater than $15 m^2/g$, scattering intensity of light having a short wavelength increases as compared with scattering intensity of light having a long wavelength, and thus paleness increases.

As a method of measuring the BET specific surface area, for example, a method of performing measurement from a nitrogen adsorption isotherm by the BET multipoint method using a fully automated specific surface area measuring device (trade name: BELSORP-Mini II, manufactured by MicrotracBEL Corp.) is mentioned.

[Titanium oxide particles]

The titanium oxide powder of the present embodiment is an agglomerate of titanium oxide particles.

A shape of each of the titanium oxide particles of the present embodiment is an octahedral shape in which an average value of the maximum values of the line segments each of which connects two apexes which face each other is 300 nm or higher and 1000 nm or lower.

Light can be scattered over a wide range by making the shape of each of the titanium oxide particles an octahedral shape in which an average value of the maximum values of the line segments each of which connects two apexes which face each other is 300 nm or higher and 1000 nm or lower. Therefore, when the cosmetics containing the titanium oxide powder is applied to the skin, a feeling of transparency and a concealing ability can be improved.

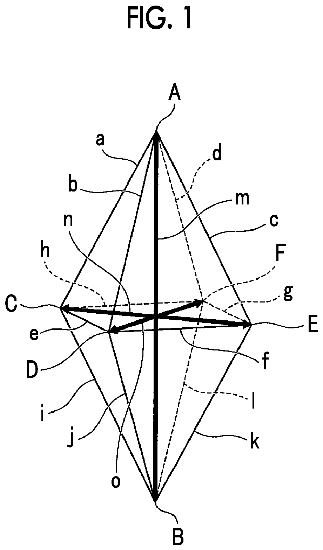

An octahedral shape is a three-dimensional shape in which an internal space is surrounded by eight triangles, as shown in FIG. 1. The eight triangles may all have the same shape, or may have two or more different shapes including shapes of two different types. A tip end part of the respective apexes of the octahedral-shaped titanium oxide particles (points indicated by reference signs A, B, C, D, E, and F in FIG. 1) may have a sharp shape, a rounded shape, or a flat shape.

A content of octahedral-shaped titanium oxide particles in the titanium oxide powder, in which an average value obtained from the maximum values of the line segments each of which connects two apexes which face each other is 300 nm or higher and 1000 nm or lower, is represented by % by number calculated by a method described later. That is, the content of octahedral-shaped titanium oxide particles in the titanium oxide powder is preferably 50% by number or more, may be 60% by number or more, or may be 70% by number or more. The upper limit of the content of octahedral-shaped titanium oxide particles in the titanium oxide powder may be 80% by number, may be 90% by number, or may be 100% by number.

A case where the content of octahedral-shaped titanium oxide particles in the titanium oxide powder is 50% by number or more is preferable from the viewpoint of further decreasing paleness peculiar to titanium oxide particles while having an excellent concealing ability and a feeling of transparency in a case where cosmetics containing titanium oxide powder are applied to the skin.

The content of octahedral-shaped titanium oxide particles in the titanium oxide powder, that is, % by number can be calculated by, for example, observing 100 titanium oxide particles with a scanning electron microscope (SEM) and counting the number of octahedral-shaped titanium oxide particles, which are included in the 100 particles.

(Line segment which connects two apexes which face each other)

Each particle of the octahedral-shaped particles has line segments each of which connects two apexes which face each other (hereinafter, referred to as "distances between apexes" or "distances between two apexes which face each other" in some cases) and has a maximum value of the line segments, and an average value of the maximum values is 300 nm or higher and 1000 nm or lower, preferably 320 nm or higher and 900 nm or lower, more preferably 330 nm or higher and 800 nm or lower, and even more preferably 340 nm or higher and 750 nm or lower.

The average value of the maximum values means an average value obtained from a plurality of octahedral-shaped titanium oxide particles wherein each octahedral-shaped titanium oxide particle thereof has a plurality of line segments each of which connects two apexes which face each other and has a maximum value of lengths of the longest line segments. In addition, the line segment connecting two apexes which face each other exists inside the particle but not a surface of the particle.

The octahedral-shaped particle, which has distances between two apexes which face each other and has a maximum value of the distances and in which an average value of the maximum values is 300 nm or higher and 1000 nm or lower, is capable of scattering visible light over a wide range as compared with spherical-shaped titanium oxide particles and spindle-shaped titanium oxide particles. Therefore, it is presumed that cosmetics which contain a titanium oxide powder including the octahedral-shaped particles are capable of decreasing paleness peculiar to titanium oxide particles while achieving both a concealing ability and a feeling of transparency.

A case where the octahedral-shaped particle has distances between two apexes which face each other and has a maximum value of the distances, and an average value of the maximum values is 300 nm or higher and 1000 nm or lower is advantageous from the viewpoint of further decreasing paleness peculiar to titanium oxide particles while having an excellent feeling of transparency in a case of being applied to the skin.

In a case where the octahedral-shaped particle has distances between two apexes which face each other and has a maximum value of the distances, and an average value of the maximum values is 300 nm or higher, light having a short wavelength is hardly scattered and a pale color is suppressed, that is, paleness peculiar to titanium oxide particles can be decreased, which is preferable. On the other hand, in a case where each particle of the octahedral-shaped particles has distances between two apexes which face each other and has a maximum value of the distances, and an average value of the maximum values is 1000 nm or lower, an excellent feeling of transparency is obtained, which is preferable.

A maximum value of distances between two apexes which face each other in the octahedral-shaped particle is measured by observing the octahedral-shaped particles with a scanning electron microscope (SEM). Specifically, with respect to 100 octahedral-shaped particles, the maximum value of the distances between two apexes which face each other in each particle is measured, and the value obtained by arithmetically averaging the plurality of obtained measured values is an average value of the maximum values of the distances between two apexes which face each other.

Here, in a case where the octahedral-shaped particles are not agglomerated with one another as in the present invention, distances between apexes in one octahedral-shaped particle, that is, distances between apexes in a primary particle are measured. On the other hand, in a case of comparing the embodiment of the present invention with other cases in which particles are agglomerated with one another to form agglomerated particles, distances between apexes in the agglomerated particles, that is, distances between apexes in a secondary particle are measured. As examples of the agglomerated particles, agglomerated particles in which particles are agglomerated with one another to form octahedral-shaped agglomerates, agglomerated particles in which octahedral-shaped particles are agglomerated with one another, and the like are mentioned. The same applies to the measurement of the maximum value (X) and the minimum value (Y) described later.

In a case where a tip end part of the apexes of the octahedral-shaped particles is a flat surface, the maximum value of the distances between two apexes which face each other is measured by using a center point of the flat surface as the apex.

The octahedral-shaped particle has line segments each of which connects two apexes which face each other and has a maximum value of lengths of the line segments, and the maximum value, for example, the maximum value of lengths of the line segments (a long axis m of the octahedral-shaped particle shown in FIG. 1) each of which connects two apexes which face each other (points A and B shown in FIG. 1) is denoted by X (nm). On the other hand, a minimum value of lengths of the line segments each of which connects two apexes which face each other, for example, a minimum value of lengths of line segments (a long axis m of the octahedral-shaped particle shown in FIG. 1) each of which is orthogonal to or substantially orthogonal to the line segments (a long axis m of the octahedral-shaped particle shown in FIG. 1) related with the maximum value and each of which connects two apexes which face each other (points C and E or points D and F shown in FIG. 1) in the octahedral-shaped particle is denoted by Y (nm). At this time, an average value of ratios of X to Y (X/Y) is preferably 1.5 or higher and 3.0 or lower, and more preferably 1.5 or higher and 2.5 or lower.

A case where the average value of the ratios (X/Y) is 1.5 or higher and 3.0 or lower is advantageous from the viewpoint that cosmetics which contain a titanium oxide powder including the octahedral-shaped particles are capable of obtaining a light scattering effect of the octahedral-shaped particles in a more effective manner and of further improving a feeling of transparency in a case of being applied to the skin.

Being substantially orthogonal as described above indicates that two line segments (a long axis and a short axis of the octahedral-shaped particles) intersect at an angle of 70° to 90°. In addition, regarding being substantially orthogonal as described above, two line segments (the long axis and the short axis of the octahedral-shaped particles) may be close to each other and intersect each other, or two line segments (the long axis and the short axis of the octahedral-shaped particles) may not necessarily have an intersection point.

An octahedral shape is preferably a shape of a rectangular bipyramid in which a rectangular bottom surface is shared by two rectangular pyramids. The octahedral shape in the present embodiment is preferably a shape in which two congruent rectangular pyramids share a rectangular bottom surface, and more preferably a shape in which two congruent rectangular pyramids share a square bottom surface. A tip end part of the rectangular bipyramid may have a sharp shape, a rounded shape, or a flat shape. In addition, in an example of the present embodiment, a side surface shape of the rectangular pyramid is an isosceles triangle, not an equilateral triangle. It is preferable that the side surface shape is an isosceles triangle. The maximum value (X) of distances between two apexes which face each other in the octahedral-shaped particle means a length of a line segment that gives a distance between two apexes present in a direction orthogonal with respect to the bottom surface of the rectangular pyramids. In addition, the minimum value (Y) of distances between two apexes which face each other in the octahedral-shaped particle means a length of a shorter diagonal line in two diagonal lines on the bottom surface of the two rectangular pyramids.

Here, the distance between two apexes will be described with reference to the drawings. FIG. 1 is a schematic diagram showing a preferred example of octahedral-shaped titanium oxide particles in the titanium oxide particles of the present embodiment. With regard to a plurality of apexes included in each particle of the octahedral-shaped particles, as distances between two apexes, there are a total 15 distances of a distance a between points A and C, a distance b between points A and D, a distance c between points A and E, a distance d between points A and F, a distance e between points C and D, a distance f between the points D and E, a distance g between points E and F, a distance h between points F and C, a distance i between points B and C, a distance j between points B and D, a distance k between points B and E, a distance 1 between points B and F, a distance n between points C and E, a distance o between points D and F, and a distance m between points A and B, as shown in FIG. 1. In FIG. 1, as distances between two apexes which face each other in the octahedral-shaped particle, there are 3 distances of the distance n between points C and E, the distance o between points D and F, and the distance m between points A and B. A maximum value of the distances between two apexes which face each other in the octahedral-shaped particle is the distance m, which corresponds to the maximum value (X) of the distances between two apexes which face each other in the octahedral-shaped particle. In addition, in FIG. 1, line segments each of which is substantially orthogonal to a line segment which corresponds to the maximum value X, and each of which connects two apexes which face each other in the octahedral-shaped particle are the distance n and the distance o. In the distance n and the distance o, a shorter distance corresponds to the minimum value (Y) of the distances between two apexes which face each other in the octahedral-shaped particle.

The maximum value (X) (nm) of the distances between two apexes which face each other in the octahedral-shaped particle, and the minimum value (Y) (nm) of the distances between two apexes which face each other in the octahedral-shaped particle can be measured, for example, by observing the octahedral-shaped particles using a scanning electron microscope (SEM).

The above ratio (X/Y) is calculated by observing the titanium oxide particles with a scanning electron microscope (SEM) and measuring the maximum value (X) and the minimum value (Y). The value obtained by calculating the ratios (X/Y) for the 100 octahedral-shaped titanium oxide particles, respectively, and arithmetically averaging the obtained plurality of values is an average value of the above ratios (X/Y).

(D90/D10)

A maximum value of line segments (distances between apexes) each of which connects two apexes which face each other in one octahedral-shaped particle is obtained for a plurality of octahedral-shaped particles. A value (D90/D10) obtained by dividing the value (D90) in a case where the cumulative number percentage is 90% by the value (D10) in a case where the cumulative number percentage is 10% is 1 or higher and 3 or lower. D90/D10 may be in this range. D90/D10 is preferably 1.0 to 3.0, and D90/D10 may be 1.0 to 2.8, 1.3 to 2.5, 1.5 to 3.0, 1.5 to 2.4, 1.8 to 2.8, or the like, as necessary.

The cumulative number percentage is a cumulative percentage based on the number. For example, in a graph, the horizontal axis represents maximum value sizes (lengths) in order from smallest to largest, and the vertical axis represents cumulative%. The number of particles (ratio of the number) of each class (size of each maximum value) on the horizontal axis is cumulated. As a result, it is possible to obtain a size having the maximum value (D10, D90, or the like) in the required cumulative% (for example, 10% or 90%).

A case where D90/D10 of the distance between the apexes is within the above range represents that the sizes of the octahedral-shaped particles are almost uniform. D90/D10 is 1 or higher and 3 or lower, whereby a feeling of squeaky is suppressed in a case where the cosmetics containing the titanium oxide powder of the present embodiment are applied to the skin.

Theoretically, D90/D10 not becomes lower than 1. In a case where D90/D10 is higher than 3, a feeling of squeaky in a case of being applied to the skin increases, which is not preferable.

D10 means a value of maximum values in a cumulative 10% and D90 means a value of maximum values in a cumulative 90%, in a case where each of the maximum values of the distances between two apexes which face each other in the plurality of octahedral-shaped particles is measured, measurement values thereof are arranged in order from smallest, and the number of particles (ratios of the number of particles) is cumulated.

For example, with respect to 100 octahedral-shaped particles, the maximum value of the distances between two apexes which face each other is measured with a scanning electron microscope. These measurement results are arranged in order from smallest, and the number of particles (ratios of the number of particles) is cumulated. D10 represents the 10th value of the measurement results counting them from the smallest. D90 represents the 90th value of the measurement results counting them from the smallest.

(Average particle diameter converted from BET specific surface area)

An average particle diameter (hereinafter also referred to as "BET-converted average particle diameter") of the titanium oxide powder which is converted from a BET specific surface area of the titanium oxide powder is preferably 300 nm or higher and 1000 nm or lower, more preferably 310 nm or higher and 800 nm or lower, and even more preferably 320 nm or higher and 700 nm or lower.

The BET-converted average particle diameter of the titanium oxide powder can be calculated by Expression (1) in a case where the titanium oxide particles have an octahedral shape.

$$\text{BET-converted average particle diameter (nm)} =$$

$$16240/(\text{BET specific surface area } (m^2/g) \times \rho \ (g/cm^3)) \ ... \ (1)$$

In Expression (1), $\rho$ represents a density of the titanium oxide.

Even in a case where the titanium oxide powder contains titanium oxide particles having a shape other than octahedral-shaped particles, the BET-converted average particle diameter is calculated by using Expression (1) in a case where the octahedral-shaped particles of 50% by number or more are contained in the titanium oxide powder.

(Average value of maximum values/BET-converted average particle diameter)

A value (average value of maximum values/BET-converted average particle diameter), which is obtained by dividing the average value of the maximum values by the average particle diameter of the octahedral-shaped particles which is converted from the BET specific surface area, is preferably 0.5 or higher and 2.5 or lower, more preferably 0.7 or higher and 1.4 or lower, and even more preferably 0.9 or higher and 1.3 or lower.

In a case where (the average value of the maximum values/the BET-converted average particle diameter) is lower than 0.5, it is assumed that fine pores and the like are present in the titanium oxide particles, a refractive index as particles is lower than an original value of titanium oxide particles, which may, as a result, decrease a concealing ability. On the other hand, in a case where (the average value of the maximum values/the BET-converted average particle diameter) is 2.5 or lower, in a case of applying cosmetics which contain the titanium oxide powder to the skin, it is possible to obtain a light scattering effect due to a shape of the titanium oxide particles and thus it is possible to improve a feeling of transparency.

In general, in a case where the octahedral-shaped particles do not agglomerate with one another, the average particle diameter of the octahedral-shaped particles which is converted from the BET specific surface area is measured by making an observation with an electron microscope, and roughly matches an arithmetic average value of maximum values, the maximum value being a maximum value of line segments each of which connects two apexes which face each other in the octahedral-shaped particle.

Therefore, the fact that the value of (the average value of the maximum values/the BET-converted average particle diameter) is closer to 1.0 means that the titanium oxide particles are less likely to agglomerate with one another and more particles are present in a state of primary particles.

On the other hand, in a case where primary particles agglomerate with one another to form the octahedral-shaped particles, an arithmetic average value of maximum values which are measured by making an observation with an electron microscope and in which the maximum value is a maximum value of line segments each of which connects two apexes which face each other in the octahedral-shaped particle, does not match the average particle diameter of the octahedral-shaped particles which is converted from the BET specific surface area. Therefore, in a case where the primary particles agglomerate with one another to form the octahedral-shaped particles, the average value of the maximum values/the BET-converted average particle diameter is higher than 2.5.

(Crystalline phase)

A crystalline phase (a crystalline structure) of the titanium oxide powder of the present embodiment is not particularly limited, and may be any one single phase of an anatase type, a rutile type, and a brookite type, or may be a mixed phase thereof. Among these, the crystalline phase of the titanium oxide powder of the present embodiment is preferably the anatase type.

A case where the crystalline phase of the titanium oxide powder is the anatase type is advantageous from the viewpoint that a concealing ability is further increased in a case where cosmetics containing the titanium oxide powder is applied to the skin, and a color which is close to a color of the human skin is obtained in a case of being mixed with a cosmetic base.

The fact that the titanium oxide powder has the anatase type can be confirmed by, for example, an X-ray diffractometer (trade name: X'Pert PRO, manufactured by Spectris Co., Ltd.). In a case where a measurement result by the X-ray diffractometer is an anatase single phase, a titanium oxide powder has the anatase type.

(Surface treatment)

The titanium oxide powder or the titanium oxide particles of the present embodiment may have an inorganic compound or an organic compound on a surface thereof.

As a method of attaching the inorganic compound or the organic compound to the surface of the titanium oxide particles, for example, a method of performing a surface treatment using a surface treatment agent, and the like are mentioned.

The surface treatment agent is not particularly limited as long as the surface treatment agent can be used in cosmetics, and can be appropriately selected depending on a purpose. As the surface treatment agent, an inorganic component and an organic component are mentioned.

As the inorganic component, silica, alumina, and the like are mentioned.

As the organic component, for example, a silicone compound, an organopolysiloxane, a fatty acid, fatty acid soap, fatty acid ester, an organic titanate compound, a surfactant, a non-silicone compound, and the like are mentioned. One of these may be used alone, or two or more thereof may be used in combination.

As the silicone compound, for example, silicone oil such as methyl hydrogen polysiloxane, dimethyl polysiloxane, and methylphenyl polysiloxane; alkyl silane such as methyl trimethoxysilane, ethyl trimethoxysilane, hexyl trimethoxysilane, and octyl trimethoxysilane; fluoroalkyl silane such as trifluoromethylethyl trimethoxysilane and heptadecafluorodecyl trimethoxysilane; methicone, hydrogen dimethicone, triethoxysilyl ethyl polydimethylsiloxyethyl dimethicone, triethoxysilylethyl polydimethylsiloxyethylhexyl dimethicone, (acrylates/acrylic acid tridecyl/triethoxysilylpropyl methacrylate/dimethicone methacrylate) copolymer, triethoxycaprylyl silane, and the like are mentioned. In addition, the silicone compound may be a monomer of a compound or a copolymer thereof. One of these may be used alone, or two or more thereof may be used in combination.

As the fatty acid, for example, palmitic acid, isostearic acid, stearic acid, lauric acid, myristic acid, behenic acid, oleic acid, rosin acid, 12-hydroxystearic acid, and the like are mentioned.

As the fatty acid soap, for example, aluminum stearate, calcium stearate, aluminum 12-hydroxystearate, and the like are mentioned.

As the fatty acid ester, for example, dextrin fatty acid ester, cholesterol fatty acid ester, sucrose fatty acid ester, starch fatty acid ester, and the like are mentioned.

As the organic titanate compound, for example, isopropyl triisostearoyl titanate, isopropyl dimethacryl isostearoyl titanate, isopropyl tri(dodecyl) benzene sulfonyl titanate, neopentyl (diallyl)oxy-tri(dioctyl) phosphate titanate, neopentyl (diallyl)oxy-trineododecanoyl titanate, and the like are mentioned.

According to the titanium oxide powder of the present embodiment, in a case where cosmetics containing the titanium oxide powder are applied to the skin, it is possible to obtain natural finish in which paleness peculiar to titanium oxide particles is decreased while achieving both a concealing ability and a feeling of transparency. In addition, a feeling of squeaky is suppressed. Furthermore, in a case where the cosmetics containing the titanium oxide powder of the present embodiment are applied to the skin, spreadability and adhesion to the skin are excellent.

Therefore, the titanium oxide powder of the present embodiment can be suitably used for cosmetics, and, in particular, can be suitably used for base makeup cosmetics.

[Method for manufacturing titanium oxide powder]

A method for manufacturing a titanium oxide powder of the present invention has a first step of preparing a reaction solution by mixing a hydrolyzed product of a titanium alkoxide or a titanium metal salt with a compound having a five-membered ring that contains nitrogen, and subjecting this reaction solution to hydrothermal synthesis, to produce titanium oxide particles. In addition, the method for manufacturing a titanium oxide powder of the present invention has a second step of mixing a reaction solution containing the titanium oxide particles which have been subjected to hydrothermal synthesis and are obtained in the first step with the same reaction solution as in the first step which have not been subjected to hydrothermal synthesis, in a predetermined range, and subjecting the mixture to hydrothermal synthesis. Furthermore, the method for manufacturing a titanium oxide powder of the present invention has a third step of drying the solution obtained in the second step at 400°C or lower.

(First step)

The first step is a step of producing titanium oxide particles.

In the first step, the hydrolyzed product of titanium alkoxide or titanium metal salt is mixed with the compound having a five-membered ring that contains nitrogen to prepare a reaction solution, and this reaction solution is subjected to hydrothermal synthesis to produce titanium oxide particles.

(Hydrolyzed product of titanium alkoxide or titanium metal salt)

The hydrolyzed product of titanium alkoxide or titanium metal salt is obtained by hydrolyzing the titanium alkoxide or the titanium metal salt.

The hydrolyzed product is, for example, a cake-like solid which is a white solid, and is hydrated titanium oxide called metatitanic acid or orthotitanic acid.

As the titanium alkoxide, for example, tetraethoxytitanium, tetraisopropoxytitanium, tetra-n-propoxytitanium, tetra-n-butoxytitanium, and the like are mentioned. One of these may be used alone, or two or more thereof may be used in combination. Among these, tetraisopropoxytitanium and tetra-n-butoxytitanium are preferable, and tetraisopropoxytitanium is more preferable, from the viewpoint of easy availability and easy control of a hydrolysis rate.

As the titanium metal salt, for example, titanium tetrachloride, titanium sulfate, and the like are mentioned. One of these may be used alone, or two or more thereof may be used in combination.

In the present embodiment, in order to obtain high-purity anatase type titanium oxide particles, it is preferable to use a high purity titanium alkoxide or a high purity titanium metal salt.

The hydrolyzed product contains a by-product such as alcohols, hydrochloric acid, and sulfuric acid.

Since the by-product inhibits nucleation and crystal growth of titanium oxide particles, it is preferable to clean the hydrolyzed product with pure water.

As a method for cleaning the hydrolyzed product, for example, decantation, Nutsche method, ultrafiltration method, and the like are mentioned.

(Compound having five-membered ring that contains nitrogen)

The compound having a five-membered ring that contains nitrogen is contained in the reaction solution due to a function as a pH adjuster of the reaction solution and a function as a catalyst for hydrothermal synthesis.

As the compound having a five-membered ring that contains nitrogen, for example, pyrrole, imidazole, indole, purine, pyrrolidine, pyrazole, triazole, tetrazole, isothiazole, isoxazole, furazan, carbazole, 1,5-diazabicyclo-[4.3.0]-5-nonene, and the like are mentioned. One of these may be used alone, or two or more thereof may be used in combination.

Among these, as the compound having a five-membered ring that contains nitrogen, a compound containing one nitrogen atom is preferable from the viewpoint of narrowing a particle size distribution of titanium oxide powder and of further improving crystallinity. For example, pyrrole, indole, pyrrolidine, isothiazole, isoxazole, furazan, carbazole, and 1,5-diazabicyclo-[4.3.0]-5-nonene are preferable.

Among these, as the compound having a five-membered ring that contains nitrogen, a compound which contains one nitrogen atom and of which a five-membered ring has a saturated heterocyclic structure is preferable from the viewpoint of narrowing a particle size distribution of titanium oxide powder and of further improving crystallinity, and, for example, pyrrolidine, and 1,5-diazabicyclo-[4.3.0]-5-nonene are more preferable.

A method for preparing the reaction solution is not particularly limited, and can be appropriately selected depending on a purpose. For example, a method of mixing by using a stirrer, a bead mill, a ball mill, an attritor, a dissolver, or the like, and the like are mentioned.

In addition, water may be added to the reaction solution so that a concentration of the reaction solution is adjusted. As the water to be added to the reaction solution, deionized water, distilled water, pure water, and the like are mentioned.

A pH of the reaction solution is preferably 9 or more and 13 or less, and more preferably 11 or more and 13 or less, from the viewpoint that a catalytic action of the compound having a five-membered ring that contains nitrogen appropriately functions and a nucleation rate becomes appropriate.

In a case where the pH of the reaction solution is in a range of 9 or more and 13 or less, production of titanium oxide particles and efficiency of crystal growth become better.

The pH of the reaction solution can be regulated by controlling a content of the compound having a five-membered ring that contains nitrogen.

A titanium atom concentration in the reaction solution can be appropriately selected depending on a size of target titanium oxide particles, and the titanium atom concentration is preferably 0.05 mol/L or more and 3.0 mol/L or less, and more preferably 0.5 mol/L or more and 2.5 mol/L or less.

In a case where the titanium atom concentration in the reaction solution is 0.05 mol/L or more and 3.0 mol/L or less, a nucleation rate becomes appropriate, so that production of titanium oxide particles and efficiency of crystal growth become better.

The titanium atom concentration in the reaction solution can be regulated by controlling a content of the hydrolyzed product of titanium alkoxide or titanium metal salt.

A molar ratio (titanium atom:compound having a five-membered ring that contains nitrogen) of titanium atoms to compounds having a five-membered ring that contains nitrogen in the reaction solution is preferably 1.0:0.5 to 1.0:2.0, and more preferably 1.0:0.6 to 1.0:1.8, and even more preferably 1.0:0.7 to 1.0:1.5.

In a case where the molar ratio of titanium atoms to compounds having a five-membered ring that contains nitrogen in the reaction solution is within the above-mentioned range, it is possible to produce octahedral-shaped titanium oxide particles.

Hydrothermal synthesis is a method in which a reaction solution is heated to allow titanium in the reaction solution to react in the presence of high-temperature and high-pressure hot water. A hydrothermal synthetic reaction is preferably carried out in a sealed container that can withstand high-temperature and high-pressure.

The hydrothermal synthesis is carried out by placing a reaction solution in a high-temperature and high-pressure container called an autoclave, sealing the autoclave, and heating the reaction solution together with the autoclave.

In a case where the reaction solution is heated, a pressure in the container rises due to evaporation of moisture in the reaction solution, which allows a high-temperature and high-pressure reaction to occur.

A heating and holding temperature in the hydrothermal synthesis is preferably 150°C or more and 350°C or less, and more preferably 150°C or more and 210°C or less.

In a case where the heating and holding temperature in hydrothermal synthesis is within the above-mentioned range, the hydrolyzed product of titanium alkoxide or titanium metal salt can have an improved solubility in water and can be dissolved in the reaction solution. In addition, the above case allows nuclei of titanium oxide particles to be produced and allows the nuclei to be grown, so that titanium oxide particles of a desired shape can be manufactured.

A heating rate in the hydrothermal synthesis is not particularly limited, and can be appropriately selected depending on a purpose.

A pressure in the hydrothermal synthesis is a pressure in a case where the reaction solution is heated to the above-mentioned temperature range in a high-temperature and high-pressure container.

During heating in the autoclave, it is preferable to stir the reaction solution using a stirring device.

A stirring speed is not particularly limited, and can be appropriately selected depending on a purpose. The stirring speed is preferably 100 rpm or more and 300 rpm or less.

A heating and holding time in the hydrothermal synthesis is not particularly limited, and can be appropriately selected depending on a size of the titanium oxide particles to be produced. The heating and holding time is preferably 3 hours or longer, and more preferably 4 hours or longer.

In a case where the heating and holding time is shorter than 3 hours, the hydrolyzed product of titanium alkoxide or titanium metal salt as a raw material may not react and a yield may decrease.

The heating and holding time is influenced by a type and a concentration of the raw material. Therefore, an appropriate preliminary experiment may be conducted, so that the hydrothermal synthesis is carried out for a heating and holding time which allows titanium oxide particles to have a desired size. For example, the heating and holding time may be 9 hours, 12 hours, 24 hours, 48 hours, or 72 hours. However, from the viewpoint of production efficiency, heating may be stopped at a time point where titanium oxide particles reach a desired size.

(Second step)

The second step is a step of further crystal-growing the titanium oxide particles obtained in the first step.

The second step is a step of mixing a reaction solution containing the titanium oxide particles which have been subjected to hydrothermal synthesis and are obtained in the first step with the same reaction solution (the hydrolyzed product of titanium alkoxide or titanium metal salt, and the compound having a five-membered ring that contains nitrogen) as in the first step which have not been subjected to hydrothermal synthesis, in a predetermined range, and subjecting the mixture to hydrothermal synthesis.

A mixing ratio of the reaction solution containing the titanium oxide particles which have been subjected to hydrothermal synthesis and are obtained in the first step to the same reaction solution (the hydrolyzed product of titanium alkoxide or titanium metal salt, and the compound having a five-membered ring that contains nitrogen) as in the first step which have not been subjected to hydrothermal synthesis is preferably 1:0.5 to 1:5, and more preferably 1:0.6 to 1:3 in a case of being converted by mass of titanium oxide particles.

A hydrothermal synthesis in the second step can be carried out under the same conditions as in the first step.

In a case of growing the titanium oxide particles obtained in the second step, a reaction solution containing the titanium oxide particles which have been subjected to hydrothermal synthesis and are obtained in the second step may be mixed with the same reaction solution (the hydrolyzed product of titanium alkoxide or titanium metal salt, and the compound having a five-membered ring that contains nitrogen) as in the first step which have not been subjected to hydrothermal synthesis, with the same mixing ratio as described above, and then may be subjected to hydrothermal synthesis under the same conditions as in the first step. In addition, the same steps may be repeated one or more times until titanium oxide particles having a desired size are obtained.

(Third step)

A method of taking out the titanium oxide powder from the reaction solution after carrying out the second step is not particularly limited, and can be appropriately selected depending on a purpose. As the method of taking out the titanium oxide from the reaction solution, for example, a method of performing solid-liquid separation such as decantation and Nutsche method, and the like are mentioned.

After taking out the titanium oxide powder, the obtained titanium oxide powder may be cleaned with pure water or the like for the purpose of decreasing impurities.

The titanium oxide powder taken out by solid-liquid separation may be naturally dried, or may be heated at 400°C or lower and dried.

The lower limit of the heating temperature of the titanium oxide powder is not particularly limited as long as the titanium oxide powder can be dried. For example, the heating temperature may be 200°C, may be 250°C, or may be 300°C.

By drying the titanium oxide powder at 400°C or lower, it is possible to obtain a titanium oxide powder having excellent spreadability and adhesion to the skin in a case of being applied to the skin.

In a case where the drying temperature is high, the titanium oxide particles are fused with one another and the texture in a case of being applied to the skin is deteriorated, which is not preferable.

The titanium oxide powder of the present embodiment can be obtained by taking out the titanium oxide powder from the reaction solution which has been subjected to hydrothermal synthesis and drying the titanium oxide powder. The obtained titanium oxide powder can be stored by a preferred method selected as necessary.

It is also possible to subject the titanium oxide powder to a surface treatment. A timing of performing the surface treatment is not particularly limited, and can be appropriately selected depending on a purpose. As the timing of performing the surface treatment, for example, after the second step, after the third step, and the like are mentioned.

A method of performing the surface treatment is not particularly limited, and a known method can be appropriately selected depending on a type of a surface treatment agent to be used.

[Dispersion]

The dispersion of the present embodiment contains the titanium oxide powder of the present embodiment and a dispersion medium. The dispersion of the present embodiment contains other components as necessary.

The dispersion of the present embodiment may be in a low-viscosity liquid state or a high-viscosity paste state.

A content of the titanium oxide powder in the dispersion of the present embodiment is not particularly limited, and can be appropriately selected depending on a purpose. For example, the content of the titanium oxide powder may be 0.1% by mass or more and 90% by mass or less, 1% by mass or more and 80% by mass or less, 5% by mass or more and 70% by mass or less, 10% by mass or more and 60% by mass or less, or 20% by mass or more and 50% by mass or less, with respect to a total amount of the dispersion of the present embodiment.

(Dispersion medium)

The dispersion medium is not particularly limited as long as the dispersion medium can be blended with cosmetics, and can be appropriately selected depending on a purpose. As the dispersion medium, for example, water, alcohols, esters, ethers, ketones, hydrocarbon, amides, polysiloxanes, modified polysiloxanes, hydrocarbon oil, ester oil, a higher fatty acid, higher alcohol, and the like are mentioned. One of these may be used alone, or two or more thereof may be used in combination.

As the alcohols, for example, methanol, ethanol, 1-propanol, 2-propanol, 1-butanol, 2-butanol, octanol, glycerin, and the like are mentioned.

As the esters, for example, ethyl acetate, butyl acetate, ethyl lactate, propylene glycol monomethyl ether acetate, propylene glycol monoethyl ether acetate, g-butyrolactone, and the like are mentioned.

As the ethers, for example, diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, diethylene glycol monoethyl ether, and the like are mentioned.

As the ketones, for example, acetone, methyl ethyl ketone, methyl isobutyl ketone, acetyl acetone, cyclohexanone, and the like are mentioned.

As the hydrocarbon, for example, aromatic hydrocarbon such as benzene, toluene, xylene, and ethylbenzene; cyclic hydrocarbon such as cyclohexane, and the like are mentioned.

As the amides, dimethylformamide, N,N-dimethylacetoacetamide, N-methylpyrrolidone, and the like are mentioned.

As the polysiloxanes, for example, chain-like polysiloxanes such as dimethylpolysiloxane, methylphenylpolysiloxane, and diphenylpolysiloxane; cyclic polysiloxanes such as octamethylcyclotetrasiloxane, decamethylcyclopentasiloxane, and dodecamethylcyclohexasiloxane, and the like are mentioned.

As the modified polysiloxanes, for example, an amino-modified polysiloxane, a polyether-modified polysiloxane, an alkyl-modified polysiloxane, a fluorine-modified polysiloxane, and the like are mentioned.

As the hydrocarbon oil, for example, liquid paraffin, squalane, isoparaffin, branched chain-like light paraffin, petrolatum, ceresin, and the like are mentioned.

As the ester oil, for example, isopropyl myristate, cetyl isooctanoate, glyceryl trioctanoate, and the like are mentioned.

As the higher fatty acid, for example, lauric acid, myristic acid, palmitic acid, stearic acid, and the like are mentioned.

As the higher alcohol, for example, lauryl alcohol, cetyl alcohol, stearyl alcohol, hexyl dodecanol, isostearyl alcohol, and the like are mentioned.

(Other components)

The other components are not particularly limited as long as the components do not impair an effect of the dispersion of the present embodiment, and can be appropriately selected depending on a purpose. As the other components, for example, a dispersant, a stabilizer, a water-soluble binder, a thickener, an oil-soluble preservative, an ultraviolet absorber, an oil-soluble agent, oil-soluble coloring matters, oil-soluble proteins, a vegetable oil, an animal oil, and the like are mentioned. One of these may be used alone, or two or more thereof may be used in combination.

A content of the dispersion medium is not particularly limited, and can be appropriately selected depending on a purpose. A content of the dispersion medium is preferably 10% by mass or more and 99% by mass or less, more preferably 20% by mass or more and 90% by mass or less, and even more preferably 30% by mass or more and 80% by mass or less, with respect to a total amount of the dispersion of the present embodiment.

According to the dispersion of the present embodiment, in a case where cosmetics containing the dispersion of the present embodiment is applied to the skin, it is possible to obtain natural finish in which paleness peculiar to titanium oxide particles is decreased while achieving both a concealing ability and a feeling of transparency. In addition, a feeling of squeaky is suppressed. Furthermore, in a case where the cosmetics containing the titanium oxide powder of the present embodiment are applied to the skin, spreadability and adhesion to the skin are excellent. Therefore, the dispersion of the present embodiment can be suitably used for cosmetics, and, in particular, can be suitably used for base makeup cosmetics.

[Method for manufacturing dispersion]

A method for manufacturing the dispersion of the present embodiment is not particularly limited, and a known method can be adopted. As the method for manufacturing the dispersion of the present embodiment, for example, a method of manufacturing a dispersion by mechanically dispersing the titanium oxide powder of the present embodiment with respect to a dispersion medium by a dispersing device, and the like are mentioned.

As the dispersing device, a stirrer, a self-revolution type mixer, a homomixer, an ultrasonic homogenizer, a sand mill, a ball mill, a roll mill, and the like are mentioned.

In a case of being applied to the skin, the dispersion of the present embodiment is capable of decreasing paleness peculiar to titanium oxide while achieving both a concealing ability and a feeling of transparency. In addition, a feeling of squeaky is suppressed. Furthermore, in a case where the cosmetics containing the titanium oxide powder of the present embodiment are applied to the skin, spreadability and adhesion to the skin are excellent.

[Cosmetics]

The cosmetics of the present embodiment contain the titanium oxide powder of the present embodiment and a cosmetic base. The cosmetics of the present embodiment contain other components as necessary.

Any content of the titanium oxide powder in the cosmetics can be selected, but the content of the titanium oxide powder is preferably 0.1% by mass or more and 50% by mass or less, with respect to a total of the cosmetics. For example, the content of the titanium oxide powder may be 0.1% to 5% by mass, 5% to 15% by mass, 15% to 35% by mass, or 35% to 50% by mass.

(Cosmetic base)

The cosmetic base can be appropriately selected from cosmetic bases usually used in cosmetics, and, for example, talc, mica, and the like are mentioned. One of these may be used alone, or two or more thereof may be used in combination.

A content of the cosmetic base in the cosmetics is not particularly limited, and can be appropriately selected depending on a purpose.

(Other components)

In addition to the titanium oxide powder and the cosmetic base of the present embodiment, the cosmetics of the present embodiment can contain other components within a range which does not impair an effect of the present embodiment.

The other components can be appropriately selected from components usually used in cosmetics. As the other components, for example, a solvent, an oil agent, a surfactant, a humectant, an organic ultraviolet absorber, an antioxidant, a thickener, a fragrance, a colorant, a physiologically active component, an antibacterial agent, and the like are mentioned. One of these may be used alone, or two or more thereof may be used in combination.

A content of the other components in the cosmetics is not particularly limited, and can be appropriately selected depending on a purpose.

A method for manufacturing the cosmetics of the present embodiment is not particularly limited, and can be appropriately selected depending on a purpose. As the method for manufacturing the cosmetics of the present embodiment, for example, a manufacturing method in which the titanium oxide powder is mixed with the cosmetic base and the mixture is mixed with the other components, a manufacturing method in which the titanium oxide powder is mixed with existing cosmetics, a manufacturing method in which the dispersion is mixed with the cosmetic base and the mixture is mixed with the other components, and a manufacturing method in which the dispersion is mixed with existing cosmetics, and the like are mentioned.

(Form)

A form of the cosmetics of the present embodiment is not particularly limited, and can be appropriately selected depending on a purpose. As the form of the cosmetics of the present embodiment, for example, a powder-like form, powdery solid-like form, a solid-like form, a liquid-like form, a gel-like form, and the like are mentioned. In a case where the form of the cosmetics is liquid-like or gel-like, a dispersion form of the cosmetics is not particularly limited, and can be appropriately selected depending on a purpose. As the dispersion form of the gel-like cosmetics, for example, a water-in-oil type (W/O type) emulsion, an oil-in-water type (O/W type) emulsion, an oil type, and the like are mentioned.

As the cosmetics of the present embodiment, for example, base makeup, nail polish, lipstick, and the like are mentioned. Among these, the base makeup is preferable.

As the base makeup, for example, makeup base used mainly for decreasing irregularities of the skin, foundation used mainly for adjusting a color of the skin, face powder used mainly for improving fixation of foundation to the skin, and the like are mentioned.

According to the cosmetics of the present embodiment, it is possible to decrease paleness peculiar to titanium oxide particles while having a concealing ability and a feeling of transparency in a case of being applied to the skin and to suppress a feeling of squeaky. Furthermore, in a case where the cosmetics of the present embodiment are applied to the skin, spreadability and adhesion to the skin are excellent.

Examples

Hereinafter, the present invention will be described more specifically with reference to Examples and Comparative Examples. However, the present invention is not limited to the following Examples.

[Example 1]

(Production of titanium oxide powder)

1 L of pure water was placed in a glass container having a capacity of 2 L, and 1 mol of tetraisopropoxytitanium (trade name: A-1, manufactured by Nippon Soda Co., Ltd.) was added dropwise while performing stirring, to obtain a white suspension containing a hydrolyzed product of a titanium alkoxide.

Next, the white suspension was subjected to solid-liquid separation, to obtain a white cake B (1 mol (80g) in terms of titanium oxide) which is a solid portion of the hydrolyzed product of titanium alkoxide.

Next, pyrrolidine (manufactured by Kanto Chemical Co., Inc.) in an amount of 0.7 mol and the obtained white cake were placed in an autoclave, and pure water was added to make a total amount 1 kg. The sealed container was held at 220°C for 9 hours and hydrothermal synthesis was carried out to obtain a reaction solution (C1) containing the titanium oxide particles.

Next, 500 g of the obtained reaction solution (C1) (40 g of titanium oxide), 0.5 mol (40 g) of the white cake B obtained in the same manner as the above described production process, in terms of titanium oxide, and 0.35 mol of pyrrolidine were placed in an autoclave. Furthermore, pure water was added to make a total amount 1 kg, the sealed container was held at 220°C for 9 hours, and hydrothermal synthesis was carried out to obtain a reaction solution (D1) containing the titanium oxide particles.

The reaction solution (D1) containing titanium oxide particles was subjected to solid-liquid separation and the solid was dried at 200°C, to obtain a titanium oxide powder of Example 1.

(Measurement of shape)

"Measurement of line segments connecting two apexes which face each other and content of octahedral-shaped particles"

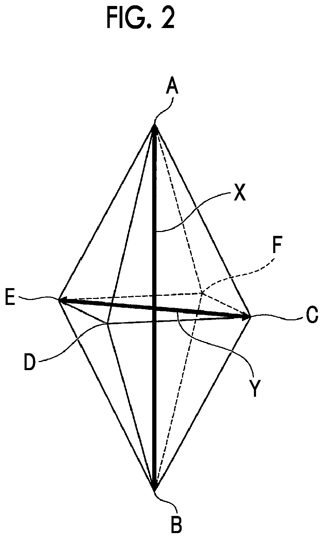

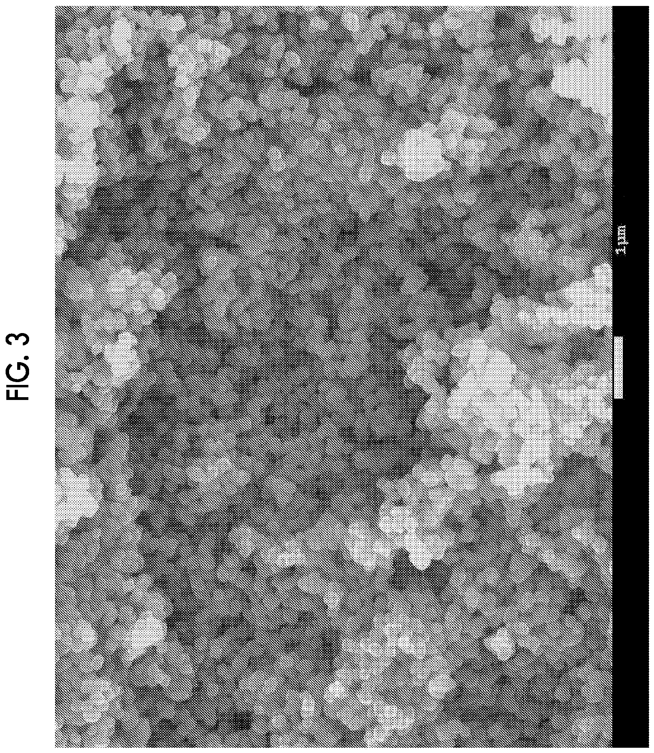

In FIG. 2, a maximum value (hereinafter indicated by (X)) of line segments each of which connects two apexes which face each other in each particle, and a minimum value (hereinafter indicated by (Y)) of line segments, each of which is substantially orthogonal to a line segment which corresponds to the maximum value, and each of which connects two apexes which face each other, were measured with a scanning electron microscope (SEM) (trade name: S-4800, manufactured by Hitachi High-Technologies Corporation) by observing a secondary electron image of the titanium oxide particles of Example 1. The SEM image is shown in FIG. 3.

100 octahedral-shaped particles included in the titanium oxide particles of Example 1 were observed, and each value of the above (X), an average value of the above (X), a value (D10) in a case where a cumulative number percentage of the above (X) was 10%, a value (D90) in a case where a cumulative number percentage of the above (X) was 90%, D90/D10, an average value of the above (Y), and an average value of ratios (X/Y) of the X to the Y were calculated. As a result, the average value of (X) was 520 nm, the D90/D10 was 2.3, and the average value of ratios (X/Y) was 2.0.

In addition, as a result of observing 100 titanium oxide particles with a scanning electron microscope, 75% by number of octahedral-shaped titanium oxide particles were present in the titanium oxide powder. The results are shown in Table 1.

(Measurement of BET specific surface area and average particle diameter converted from BET specific surface area)

A BET specific surface area of the titanium oxide powder of Example 1 was measured using a specific surface area meter (trade name: BELSORP-mini, manufactured by Bel Japan, Inc.). As a result, the BET specific surface area of the titanium oxide powder of Example 1 was 8 $m^2/g$.

An average particle diameter of the titanium oxide powder of Example 1 which is converted from a BET specific surface area was calculated by Expression (1). As a result, the BET-converted average particle diameter was 508 nm. The results are shown in Table 1.

$$\text{BET-converted average particle diameter (nm)} =$$

$$16240/(\text{BET specific surface area } (m^2/g) \times \rho \ (g/cm^3)) \ ... \ (1)$$

In Expression (1), $\rho$ represents a density of the titanium oxide, and $\rho = 4g/cm^3$.

A value (hereinafter referred to as "average value of (X)/BET-converted average particle diameter" in some cases) obtained by dividing the average value of the (X) by the BET-converted average particle diameter was 1.0.

"Identification of shape of titanium oxide particles"

The titanium oxide powder of Example 1 was observed with a transmission electron microscope (TEM) (model number: H-800, manufactured by Hitachi High-Technologies Corporation). Also in a TEM image, the octahedral-shaped titanium oxide particles were observed.

(Identification of crystalline phase of titanium oxide particles)

A crystalline phase of the titanium oxide powder of Example 1 was identified using an X-ray diffractometer (trade name: X'PertPRO, manufactured by Spectris Co., Ltd.). As a result, the titanium oxide powder of Example 1 was in an anatase single phase.

"Production of cosmetics"

2 g of the titanium oxide powder of Example 1 and 8 g of talc were mixed with each other to produce base makeup cosmetics of Example 1.

(Evaluation of paleness, feeling of transparency, and concealing ability)

Each of the base makeup cosmetics of Example 1 was applied on a 5 cm square substrate (trade name: HELIOPLATE HD-6, manufactured by Helioscreen) so as to be 12 mg to 14 mg, to produce applied substrates.

For each of the applied substrates, diffuse transmission spectrum (TT), diffuse reflection spectrum (TR), and linear reflection spectrum (R) were measured using a spectrophotometer (model number; UV-3150, manufactured by Shimadzu Corporation), and evaluation was performed using the following indices. In each case, an incident direction of light was measured from an applied surface, and the reflection spectrum was measured on the basis of a molded plate obtained by compressing barium sulfate powders (special grade, manufactured by Kanto Chemical Co., Inc.).
The results are shown in Table 1.

(Paleness)

A ratio ($TR_{450nm}/TR_{550nm}$) of a diffuse reflectance ($TR_{450nm}$) at 450 nm to a diffuse reflectance ($TR_{550nm}$) at 550 nm was used as an index for paleness. Since it can be said as being paler as the ratio becomes larger than 1, it is preferable that the value of $TR_{450nm}/TR_{550nm}$ is smaller.
A correlation between the index for paleness and an appearance viewed by human's eyes is shown in Table 2.

(Feeling of transparency)

A ratio ($R_{550nm}/TR_{550nm}$) of a linear reflectance ($R_{550nm}$) at 550 nm and the diffuse reflectance ($TR_{550nm}$) at 550 nm was used as an index for feeling of transparency. Since a smaller ratio indicates a higher feeling of transparency, it is preferable that the value is smaller.
A correlation between the index for feeling of transparency and an appearance viewed by human's eyes is shown in Table 2.

(Concealing ability)

The diffuse reflectance ($TR_{550nm}$) at 550 nm was used as an index for concealing ability. In a case where the diffuse reflectance is large, it can be said that the concealing ability is large. Thus, it is preferable that the value is large.
A correlation between the index for concealing ability and an appearance viewed by human's eyes is shown in Table 2.

(Evaluation of feeling of squeaky)

The base makeup cosmetics of Example 1 were applied to the human skin. A case where a person being applied with the base makeup cosmetics feels that there is no feeling of squeaky and feels good in use is evaluated as "o", and a case where a person being applied with the base makeup cosmetics feels that there is a feeling of squeaky and feels bad in use is evaluated as "x". "o" indicates that the evaluation is good, and "x" indicates that the evaluation is bad.
The feeling of squeaky of the base makeup cosmetics of Example 1 was "o".

[Example 2]

600 g of the reaction solution (C1) (48 g of titanium oxide) obtained in the production process of Example 1, 0.4 mol (32 g) of the white cake B obtained in the production process of Example 1, in terms of titanium oxide, and 0.28 mol of pyrrolidine were placed. Furthermore, pure water was added to make a total amount 1 kg, the container was sealed, and the sealed container was held at 220°C for 9 hours to obtain a reaction solution (D2) containing the titanium oxide particles.
The reaction solution (D2) containing titanium oxide particles was subjected to solid-liquid separation and the solid was dried at 200°C, to obtain a titanium oxide powder of Example 2.
With regard to the obtained titanium oxide powder of Example 2, a BET specific surface area, a shape, and a crystalline phase were measured in the same manner as in Example 1.
As a result, the BET specific surface area was 7 $m^2/g$, and a BET-converted average particle diameter was 580 nm. An average value of (X) in the octahedral-shaped titanium oxide particles was 600 nm, the average value of the (X)/the BET-converted average particle diameter was 1.0, D90/D10 was 1.8, and an average value of ratios (X/Y) was 2.0. The results are shown in Table 1.
In addition, in the titanium oxide particles of Example 2, octahedral-shaped titanium oxide particles were 70% by number with respect to an entirety of the particles, and a crystalline phase of the titanium oxide powder was in an anatase single phase. The results are shown in Table 1.
Base makeup cosmetics of Example 2 were obtained in the same manner as in Example 1 except that the titanium oxide powder of Example 2 was used instead of the titanium oxide powder of Example 1.
In the same manner as in Example 1, paleness, a feeling of transparency, a concealing ability, and a feeling of squeaky were evaluated. The results are shown in Table 1.

[Example 3]

400 g of the reaction solution (C1) (32 g of titanium oxide) obtained in the production process of Example 1, 0.6 mol (48 g) of the white cake B obtained in the production process of Example 1, in terms of titanium oxide, and 0.42 mol of pyrrolidine were placed. Furthermore, pure water was added to make a total amount 1 kg, the container was sealed, and the sealed container was held at 220°C for 9 hours to obtain a reaction solution (D3) containing the titanium oxide particles.

The reaction solution (D3) containing titanium oxide particles was subjected to solid-liquid separation and the solid was dried at 200°C, to obtain a titanium oxide powder of Example 3.

With regard to the obtained titanium oxide powder of Example 3, a BET specific surface area, a shape, and a crystalline phase were measured in the same manner as in Example 1.

As a result, the BET specific surface area was 9 $m^2/g$, and a BET-converted average particle diameter was 451 nm. An average value of (X) in the octahedral-shaped titanium oxide particles was 480 nm, the average value of the (X)/the BET-converted average particle diameter was 1.1, D90/D10 was 2.7, and an average value of ratios (X/Y) was 2.0. The results are shown in Table 1.

In addition, in the titanium oxide particles of Example 3, octahedral-shaped titanium oxide particles were 70% by number with respect to an entirety of the particles, and a crystalline phase of the titanium oxide powder was in an anatase single phase. The results are shown in Table 1.

Base makeup cosmetics of Example 3 were obtained in the same manner as in Example 1 except that the titanium oxide powder of Example 3 was used instead of the titanium oxide powder of Example 1.

In the same manner as in Example 1, paleness, a feeling of transparency, a concealing ability, and a feeling of squeaky of the base makeup cosmetics of Example 3 were evaluated. The results are shown in Table 1.

[Example 4]

The reaction solution (D3) obtained in the production process of Example 3 was subjected to separation at 1000 g/10 minutes by a centrifuge (Model 7930, manufactured by KUBOTA CORPORATION), and the sedimented powder was recovered. The recovered powder was dried at 200°C to obtain a titanium oxide powder of Example 4.

With regard to the obtained titanium oxide powder of Example 4, a BET specific surface area, a shape, and a crystalline phase were measured in the same manner as in Example 1.

As a result, the BET specific surface area was 8 $m^2/g$, and a BET-converted average particle diameter was 508 nm. An average value of (X) in the octahedral-shaped titanium oxide particles was 510 nm, the average value of the (X)/the BET-converted average particle diameter was 1.0, D90/D10 was 2.4, and an average value of ratios (X/Y) was 2.0. The results are shown in Table 1.

In addition, in the titanium oxide particles of Example 4, octahedral-shaped titanium oxide particles were 65% by number with respect to an entirety of the particles, and a crystalline phase of the titanium oxide powder was in an anatase single phase. The results are shown in Table 1.

Base makeup cosmetics of Example 4 were obtained in the same manner as in Example 1 except that the titanium oxide powder of Example 4 was used instead of the titanium oxide powder of Example 1.

In the same manner as in Example 1, paleness, a feeling of transparency, a concealing ability, and a feeling of squeaky of the base makeup cosmetics of Example 4 were evaluated. The results are shown in Table 1.

[Comparative Example 1]

100 g of the reaction solution (C1) (8 g of titanium oxide) obtained in the production process of Example 1, 0.9 mol (72 g) of the white cake B obtained in the production process of Example 1, in terms of titanium oxide, and 0.63 mol of pyrrolidine were placed. Furthermore, pure water was added to make a total amount 1 kg, the container was sealed, and the sealed container was held at 220°C for 9 hours to obtain a reaction solution containing the titanium oxide particles.

The reaction solution containing titanium oxide particles was subjected to solid-liquid separation and the solid was dried at 200°C, to obtain a titanium oxide powder of Comparative Example 1.

With regard to the obtained titanium oxide powder of Comparative Example 1, a BET specific surface area, a shape, and a crystalline phase were measured in the same manner as in Example 1.

As a result, the BET specific surface area was 12 $m^2/g$, and a BET-converted average particle diameter was 338 nm. An average value of (X) in the octahedral-shaped titanium oxide particles was 450 nm, the average value of the (X)/the BET-converted average particle diameter was 1.3, D90/D10 was 3.5, and an average value of ratios (X/Y) was 2.0. The results are shown in Table 1.

In addition, in the titanium oxide particles of Comparative Example 1, octahedral-shaped titanium oxide particles were 60% by number with respect to an entirety of the particles, and a crystalline phase of the titanium oxide powder was in an anatase single phase. The results are shown in Table 1.

Base makeup cosmetics of Comparative Example 1 were obtained in the same manner as in Example 1 except that the titanium oxide powder of Comparative Example 1 was used instead of the titanium oxide powder of Example 1.

In the same manner as in Example 1, paleness, a feeling of transparency, a concealing ability, and a feeling of squeaky of the base makeup cosmetics of Comparative Example 1 were evaluated. The results are shown in Table 1.

[Comparative Example 2]

Commercially available titanium oxide particles having an average diameter of 300 nm and having a spherical-shaped rutile type with a BET specific surface area of 6 $m^2$/g were used as titanium oxide particles of Comparative Example 2.

In addition, a BET-converted average particle diameter was calculated by Expression (2). Since the particles are spherical, Expression (2) different from Expression (1) was used for calculation. As a result, the BET-converted average particle diameter was 250 nm, and a value obtained by dividing the average particle diameter by the BET-converted average particle diameter was 1.2.

$$\text{BET-converted average particle diameter (nm)} =$$

$$6000/(\text{BET specific surface area (m}^2/\text{g}) \times \rho \text{ (g/cm}^3)) \ \dots \ (2)$$

In Expression (2), $\rho$ represents a density of titanium oxide, and therefore, $\rho$ = 4 g/cm$^3$. An average particle diameter of spherical-shaped particles which is converted from a BET specific surface area roughly matches an average diameter of primary particles.

In addition, in the titanium oxide particles of Comparative Example 2, an average value of (X) is 300 nm, the average value of the (X)/the BET-converted average particle diameter was 1.2, and an average value of ratios (X/Y) was 1.0. The results are shown in Table 1. Since each of the titanium oxide particles having a spherical shape, a diameter of sphere was used as the values of (X) and (Y).

In the spherical-shaped titanium oxide particles, a diameter at a predetermined position corresponds to a maximum value (X) of line segments each of which connects two apexes which face each other in each particle. In the spherical-shaped titanium oxide particles, another diameter which is substantially orthogonal to the diameter at a predetermined position corresponds to a minimum value (Y) of line segments, each of which is substantially orthogonal to a line segment which corresponds to the maximum value (X), and each of which connects two apexes which face each other.

In addition, in the titanium oxide particles of Comparative Example 2, a content of octahedral-shaped titanium oxide particles was 0% by number with respect to an entirety of the particles. The results are shown in Table 1.

Base makeup cosmetics of Comparative Example 2 were obtained in the same manner as in Example 1 except that the titanium oxide powder of Comparative Example 2 was used instead of the titanium oxide powder of Example 1.

In the same manner as in Example 1, paleness, a feeling of transparency, and a concealing ability were evaluated. The results are shown in Table 1.

[Comparative Example 3]

Commercially available titanium oxide particles were used as the titanium oxide powder of Comparative Example 3.

The titanium oxide particles, of which primary particles are a spheroid having an average major diameter of 100 nm and an average minor diameter of 30 nm and the primary particles agglomerate with one another to form a spindle shape with an average major diameter (average major diameter at agglomeration) of 300 nm, an average minor diameter (average minor diameter at agglomeration) of 136 nm, and an average value of the major diameter at agglomeration/the minor diameter at agglomeration of 2.2, and which are a rutile type with a BET specific surface area of 21 $m^2$/g, were used.

Since a primary particle shape is a spheroid, the BET-converted average particle diameter was determined using Expression (3) different from Expression (1). Specifically, P = 50 nm (100 nm/2) and Q = 3.33 (100/30) were calculated using Expression (3). As a result, the BET-converted average particle diameter was 100 nm.

$$\text{BET specific surface area } (\text{m}^2/\text{g}) = 1000 \times (1+P/((1-(1-$$
$$(1/Q)^2))^{1/2} \times P \times (1-(1/Q)^2)^{1/2} \times \sin^{-1}((1-(1/Q)^2)^{1/2}))/(2 \times \rho \times$$
$$P/3) \quad \ldots \quad (3)$$

In Expression (3), $\rho$ represents a density of the titanium oxide, and $\rho = 4\text{g/cm}^3$.

In addition, in Expression (3), P represents a radius (nm) of the average major diameter of the spheroid which is the primary particles, and Q represents an average value of aspect ratios obtained by dividing a radius of a long axis (major diameter of primary particles/2) by a radius of a short axis (minor diameter of primary particles/2).

In addition, in the titanium oxide particles of Comparative Example 3, an average value of (X) is 300 nm, the average value of the (X)/the BET-converted average particle diameter was 3.0, and an average value of ratios (X/Y) was 2.2. The results are shown in Table 1.

The spindle-shaped titanium oxide particles of Comparative Example 3 agglomerate with one another. Therefore, the average major diameter (average major diameter at agglomeration) corresponds to the maximum value (X) and the average minor diameter (average minor diameter at agglomeration) which is substantially orthogonal to a line segment which corresponds to the maximum value (X) corresponds to the minimum value (Y).

The value obtained by dividing the major diameter at agglomeration by the BET-converted average particle diameter was 3.0. The results are shown in Table 1.

Since the titanium oxide particles of Comparative Example 3 agglomerate with one another, a major diameter of an agglomerate and an average particle diameter converted from the BET specific surface area deviate from each other, and as a result, the major diameter of the agglomerate/the BET-converted average particle diameter is greater than 2.5.

In addition, in the titanium oxide particles of Comparative Example 3, a content of octahedral-shaped titanium oxide particles was 0% by number with respect to an entirety of the particles. The results are shown in Table 1.

Base makeup cosmetics of Comparative Example 3 were obtained in the same manner as in Example 1 except that the titanium oxide powder of Comparative Example 3 was used instead of the titanium oxide powder of Example 1.

In the same manner as in Example 1, paleness, a feeling of transparency, and a concealing ability were evaluated. The results are shown in Table 1.

[Table 1]

| | Example 1 | Example 2 | Example 3 | Example 4 | Comparative Example 1 | Comparative Example 2 | Comparative Example 3 |
|---|---|---|---|---|---|---|---|
| Shape | Octahedral shape | Octahedral shape | Octahedral shape | Octahedral shape | Octahedral shape | Spherical shape | Spindle shape |
| BET specific surface area (m$^2$/g) | 8 | 7 | 9 | 8 | 12 | 6 | 21 |
| BET-converted average particle diameter (nm) | 508 | 580 | 451 | 508 | 338 | 250 | 100 |
| Maximum value X (nm) | 520 | 600 | 480 | 510 | 450 | 300 | 300 |
| Maximum value (X)/ (BET-converted average particle diameter) | 1.0 | 1.0 | 1.1 | 1.0 | 1.3 | 1.2 | 3.0 |
| Maximum value (X)/ Minimum value (Y) | 2.0 | 2.0 | 2.0 | 2.0 | 2.0 | 1.0 | 2.2 |
| % by number | 75 | 70 | 70 | 65 | 60 | 0 | 0 |
| D90/D10 | 2.3 | 1.8 | 2.7 | 2.4 | 3.5 | - | - |
| Concealing ability | 52 | 55 | 50 | 52 | 50 | 48 | 42 |
| Feeling of transparency | 0.02 | 0.02 | 0.02 | 0.02 | 0.01 | 0.05 | 0.02 |
| Paleness | 1. 01 | 1. 01 | 1. 02 | 1. 01 | 1.05 | 1.03 | 1.13 |
| Feeling of squeaky | ○ | ○ | ○ | ○ | × | - | - |

EP 3 733 607 A1

[Table 2]

| | Value | Appearance viewed by human's eyes |
|---|---|---|
| Paleness $TR_{450\,nm}/TR_{550}$ nm | Lower than 1.05 | There is no paleness |
| | 1.05 or higher and lower than 1.10 | There is a little paleness |
| | 1.10 or higher | There is paleness |
| Feeling of transparency $R_{550\,nm}/TR_{550}$ nm | Lower than 0.03 | There is a feeling of transparency |
| | 0.03 or higher and lower than 0.05 | There is not much a feeling of transparency |
| | 0.05 or higher | There is no feeling of transparency |
| Concealing ability $TR_{550}$ nm | 45 or higher | There is a concealing ability |
| | 40 or higher and lower than 45 | There is not much a concealing ability |
| | Lower than 40 | There is no concealing ability |

By comparing Examples 1 to 4 with Comparative Example 1, it was confirmed that the titanium oxide powder which contains the octahedral-shaped particles having a uniform particle size, that is, having D90/D10 which was 1 or higher and 3 or lower, was excellent in a feeling of transparency and a concealing ability, and had improved paleness, and a feeling of squeaky was suppressed.

In addition, by comparing Examples 1 to 4 with Comparative Example 2 and Comparative Example 3, it was confirmed that the titanium oxide powder containing the octahedral-shaped particles which have the BET specific surface area is 5 m$^2$/g or more and 15 m$^2$/g or less and in which an average value of the maximum values of the line segments each of which connects two apexes which face each other is 300 nm or higher and 1000 nm or lower decreases paleness peculiar to titanium oxide while achieving both a concealing ability and a feeling of transparency. Therefore, it was clarified that the titanium oxide powder of the present embodiment is suitable for cosmetics for base makeup.

In order to confirm that the octahedral-shaped titanium oxide particles are capable of scattering light over a wide range, the following simulation was performed.

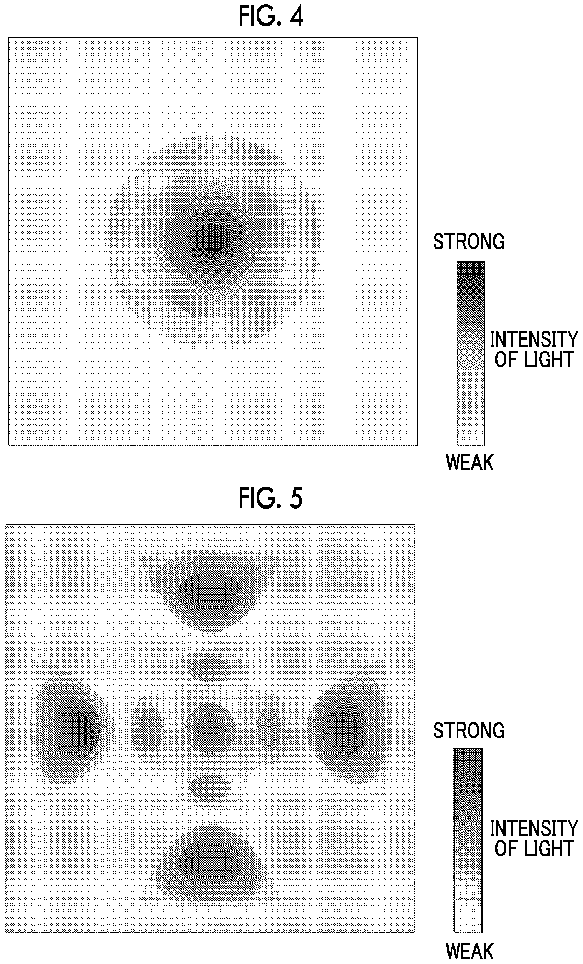

In a case where each of spherical-shaped titanium oxide particles having a diameter of 500 nm and octahedral-shaped titanium oxide particles in which a maximum value of distances between two apexes which face each other in each particle is 500 nm is irradiated with light having a wavelength of 700 nm, simulation was performed by the Finite-difference time-domain (FDTD) method on how the light scatters. The simulation results on the spherical-shaped titanium oxide particles are shown in FIG. 4. In addition, the simulation results on the octahedral-shaped titanium oxide particles are shown in FIG. 5.

In FIGS. 4 and 5, it is assumed that the titanium oxide particles are present in a center of a square-shaped display surface. Therefore, in this simulation, it can be said that a degree of scattering degree is large in a case where the light with which the titanium oxide particles present in the center are irradiated spreads larger (wider) in the display surface. On the other hand, in this simulation, it can be said that a degree of scattering is small in a case where the light with which the titanium oxide particles present in the center are irradiated does not spread or spreads small in the display surface.

From the results in FIGS. 4 and 5, it was confirmed that the octahedral-shaped titanium oxide particles scatter light up to an about 2 times longer distance than the spherical-shaped titanium oxide particles. Such results show that by making the particles an octahedral shape, it is possible to scatter light over a wide range and to achieve both a concealing ability and a feeling of transparency.

Industrial Applicability

The titanium oxide powder of the present invention has the BET specific surface area is 5 m$^2$/g or more and 15 m$^2$/g or less, and contains octahedral-shaped particles in which each particle of the octahedral-shaped particles has line segments each of which connects two apexes which face each other and has a maximum value of the line segments, and an average value of the maximum values is 300 nm or higher and 1000 nm or lower. Furthermore, since in the octahedral-shaped particles, a value obtained by dividing a value (D90) in a case where a cumulative number percentage of the maximum values of line segments each of which connects two apexes which face each other is 90% by a value (D10) in a case where a cumulative number percentage is 10% is 1 or higher and 3 or lower, it is possible to decrease paleness peculiar to titanium oxide particles while having a concealing ability and a feeling of transparency in a case of being applied to the skin and excellent in suppressing a feeling of squeaky on the skin. Therefore, the titanium oxide powder can be suitably used for base makeup cosmetics such as foundations. Furthermore, since the titanium oxide powder of the present invention has excellent performance as a white pigment, the titanium oxide powder can be used for industrial applications such as white ink and is thus industrially valuable.

Reference Signs List

X: Maximum value of line segments each of which connects two apexes which face each other
Y: Minimum value of line segment which is substantially orthogonal to a line segment which corresponds to a maximum value of distances between two apexes which face each other, and each of which connects two apexes

**Claims**

1. A titanium oxide powder, which has a BET specific surface area of 5m$^2$/g or more and 15 m$^2$/g or less, and contains octahedral-shaped particles, wherein
each octahedral-shaped particle thereof has line segments each of which connects two apexes which face each other and has a maximum value of the line segments,
an average value of the maximum values is 300 nm or higher and 1,000 nm or lower, and
a value (D90/D10) is 1 or higher and 3 or lower, wherein the value is obtained by dividing a value (D90), which corresponds to 90% in a cumulative number percentage of the maximum values of line segments each of which connects two apexes which face each other, by a value (D10) which corresponds to 10% in the cumulative number percentage.

2. The titanium oxide powder according to claim 1, wherein a value (average value of the maximum values / BET-converted average particle diameter), which is obtained by dividing the average value of the maximum values of the line segments by an average particle diameter converted from the BET specific surface area, is 0.5 or higher and 2.5 or lower.

3. The titanium oxide powder according to claim 1 or 2,
wherein a content of the octahedral-shaped particles in the titanium oxide powder is 50% by number or more.

4. The titanium oxide powder according to any one of claims 1 to 3,
wherein the titanium oxide powder has an inorganic compound or organic compound on a surface thereof.

5. A dispersion, comprising:

   the titanium oxide powder according to any one of claims 1 to 4; and
   a dispersion medium.

6. Cosmetics comprising:

   the titanium oxide powder according to any one of claims 1 to 4; and
   a cosmetic base.

7. The titanium oxide powder according to claim 1,
wherein the average value of the maximum values of the octahedral-shaped particles means an average value of maximum values of a plurality of octahedral-shaped titanium oxide particles, wherein each octahedral-shaped titanium oxide particle thereof has a plurality of line segments each of which connects two apexes which face each other and has a maximum value of lengths of the line segments.

8. The titanium oxide powder according to claim 1,
wherein the cumulative number percentage is a cumulative percentage based on number, and
the D10 represents 10th maximum value counting from a smallest measured value, and the D90 represents 90th maximum value counting from the smallest measured value, in a case where a maximum value of distances between two apexes which face each other in each particle is measured with respect to 100 octahedral-shaped particles using a scanning electron microscope, and measurement results of the maximum values of the particles are arranged in order from smallest to largest.

9. The titanium oxide powder according to claim 1,
wherein each of the octahedral-shaped particles has a shape of rectangular bipyramid in which a rectangular bottom surface is shared by two congruent rectangular pyramids, and
a shape of a tip end of the rectangular bipyramid is any one of sharp, round, or flat.

# FIG. 1

FIG. 2

FIG. 3

1μm

EP 3 733 607 A1

FIG. 4

STRONG

INTENSITY
OF LIGHT

WEAK

FIG. 5

STRONG

INTENSITY
OF LIGHT

WEAK

## INTERNATIONAL SEARCH REPORT

| International application No. |
| --- |
| PCT/JP2018/048037 |

**A. CLASSIFICATION OF SUBJECT MATTER**
Int.Cl. C01G23/053(2006.01)i, A61K8/29(2006.01)i, A61Q1/00(2006.01)i

According to International Patent Classification (IPC) or to both national classification and IPC

**B. FIELDS SEARCHED**

Minimum documentation searched (classification system followed by classification symbols)
Int.Cl. C01G23/053, A61K8/29, A61Q1/00

Documentation searched other than minimum documentation to the extent that such documents are included in the fields searched
Published examined utility model applications of Japan        1922–1996
Published unexamined utility model applications of Japan      1971–2019
Registered utility model specifications of Japan              1996–2019
Published registered utility model applications of Japan      1994–2019

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2017/115802 A1 (SUMITOMO OSAKA CEMENT CO., LTD.) 06 July 2017 & EP 3398909 A1 & FR 3046156 A1 & CN 108430926 A & KR 10-2018-0097557 A | 1-9 |
| A | JP 2007-106646 A (NATIONAL INSTITUTE OF ADVANCED INDUSTRIAL SCIENCE AND TECHNOLOGY) 26 April 2007 (Family: none) | 1-9 |
| A | JP 2008-297147 A (NATIONAL UNIVERSITY CORPORATION HOKKAIDO UNIVERSITY) 11 December 2008 (Family: none) | 1-9 |

☒ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 19 February 2019 (19.02.2019) | 26 February 2019 (26.02.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

# EP 3 733 607 A1

**INTERNATIONAL SEARCH REPORT**

| International application No. |
|---|
| PCT/JP2018/048037 |

C (Continuation). DOCUMENTS CONSIDERED TO BE RELEVANT

| Category* | Citation of document, with indication, where appropriate, of the relevant passages | Relevant to claim No. |
|---|---|---|
| A | CN 102502812 A (SHANDONG INSTITUTE OF LIGHT INDUSTRY) 20 June 2012 (Family: none) | 1-9 |
| A | CN 102849793 A (SPRING FOUNDATION OF NCTU) 02 January 2013 & TW 201300324 A | 1-9 |
| A | CN 106698503 A (CHINA PETROLEUM AND CHEMICAL CORPORATION) 24 May 2017 (Family: none) | 1-9 |
| A | US 2015/0037245 A1 (NATIONAL TSING HUA UNIVERSITY) 05 February 2015 & TW 201504154 A | 1-9 |
| A | US 2008/0156229 A1 (CORBIN, David Richard) 03 July 2008, example 4 (Family: none) | 1-9 |
| P, X | WO 2018/003851 A1 (SUMITOMO OSAKA CEMENT CO., LTD.) 04 January 2018, claims, examples (Family: none) | 1-9 |

Form PCT/ISA/210 (continuation of second sheet) (January 2015)

28

**EP 3 733 607 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017253654 A
- JP 2013028563 A
- JP 2014015340 A